Europäisches Patentamt

European Patent Office

Office européen des brevets

(19)

(11) Veröffentlichungsnummer: **0 236 575**
**B1**

(12) **EUROPÄISCHE PATENTSCHRIFT**

(45) Veröffentlichungstag der Patentschrift:
**15.11.89**

(51) Int. Cl.⁴: **C02F 1/32**, A61L 2/10

(21) Anmeldenummer: **86117868.9**

(22) Anmeldetag: **22.12.86**

(54) **Einrichtung zum Entkeimen von Flüssigkeiten.**

(30) Priorität: **12.02.86 CH 556/86**

(43) Veröffentlichungstag der Anmeldung:
**16.09.87 Patentblatt 87/38**

(45) Bekanntmachung des Hinweises auf die Patenterteilung:
**15.11.89 Patentblatt 89/46**

(84) Benannte Vertragsstaaten:
**CH DE FR GB LI**

(56) Entgegenhaltungen:
**CH-A- 636 479**
**FR-A- 631 283**
**FR-A- 2 177 012**
**FR-A- 2 452 289**
**GB-A- 651 272**
**US-A- 3 637 342**

(73) Patentinhaber: **BBC Brown Boveri AG, Haselstrasse,
CH-5401 Baden(CH)**

(72) Erfinder: **Wiesmann, Rudolf, Tulpenstrasse 6,
CH-9202 Gossau(CH)**

ACTORUM AG

## Beschreibung

Die Erfindung betrifft eine Einrichtung zum Entkeimen von Flüssigkeiten mittels ultravioletter Strahlen, mit einer Niederdruck-Hochstrom-Quecksilberdampflampe als Strahlungsquelle gemäss dem Oberbegriff des Patentanspruchs 1.

Die Erfindung nimmt damit Bezug auf einen Stand der Technik, wie er sich beispielsweise aus der CH-A 631 950 ergibt. Einrichtungen dieser Art werden in zunehmendem Mass zum Entkeimen von Industrie-, Trink- oder Schwimmbadwasser, oder auch von Getränken, Essenzen, Konzentraten oder Oelen, sofern vor allem die letztgenannten Flüssigkeiten eine ausreichende Durchlässigkeit für die ultravioletten Strahlen aufweisen, d.h. von diesen auch durchsetzt werden können. Sofern das Entladungsrohr der UV-Lampe zur Einstellung des Quecksilber-Dampfdruckes einen rohrförmigen Ansatz besass, wurde bei den bisher verwendeten Vorrichtungen der Raum, in welchem die UV-Lampe befestigt war, im wesentlichen in drei thermisch voneinander getrennte Kammern geteilt:

In eine unterste Kammer, in welcher der rohrförmige Ansatz der UV-Lampe sass, in eine mittlere Kammer, in welcher sich der Entladungsraum der Lampe befand, und welche von der zu entkeimenden Flüssigkeit umströmt wurde, und in einen oberen Teil, in welchem die Elektrodenkolben untergebracht waren, und der durch Kühlfinnen gekühlt wurde. Die Drei-Kammer-Teilung erschien deshalb notwendig, damit einerseits die Betriebstemperatur der Entladungsstrecke (i.d.R. $\geq$ 300 °C) nicht den rohrförmigen Ansatz zu sehr aufheizte, der als kälteste Stelle (i.d.R. $\leq$ 65 °C) des Systems für den Quecksilber-Dampfdruck - und damit auch für die Strahlungsintensität verantwortlich ist und andererseits auch der obere Raum durch die Entladungsstrecke nicht zu sehr aufgeheizt wurde, weil dieser von aussen zugängliche Raum aus Sicherheitsgründen nicht übermässig heiss werden durfte.

Damit die UV-Lampe überhaupt gezündet werden konnte, musste die ganze Partie um den rohrförmigen Ansatz vor dem Zünden über einen Heizwiderstand vorgewärmt werden. Diese Heizung wurde dann beim Einschalten des Anodenstromes wieder ausgeschaltet. Im Betrieb wurde die Temperatur des rohrförmigen Ansatzes durch die Rückheizung der Entladungsstrecke und die Temperatur der Umgebungsluft bestimmt. Diese wiederum schwankte mit der Wassertemperatur. Die Temperatur des rohrförmigen Ansatzes musste deshalb je nach Jahreszeit durch geeignete Zwischenringe - durch die der rohrförmige Ansatz mehr oder weniger in die unterste Kammer hineingesenkt wurde - korrigiert werden.

Die bisherigen Vorrichtungen besassen den Nachteil, dass beim Aufheizen des rohrförmigen Ansatzes zur Zündung, der mittlere Teil - und damit die Entladungsstrecke - kalt blieb. Das Quecksilber konnte daher dort wieder kondensieren, was die Zündung erschwerte. Andererseits wirkte sich die hohe Temperatur der Entladungsstrecke in der mittleren Kammer während des Betriebs ausserordentlich schädlich auf die Lebensdauer der UV-Lampe aus.

Bei der eingangs genannten CH-A 631 950 wird als Strahlungsquelle eine Niederdruck-Hochstrom-Quecksilberdampflampe verwendet, die mit einem am Entladungsrohr angeordneten rohrförmigen Ansatz versehen ist. Um die Temperaturen des rohrförmigen Ansatzes auch während des Betriebes der UV-Lampe definiert regeln zu können und um grössere Lebensdauer der UV-Lampe zu erreichen, wird zur Kühlung der UV-Lampe ein durch die Vorrichtung zirkulierender Luftstrom verwendet. Dieser Luftstrom wird derart geführt, dass die an dem Entladungsrohr erwärmte Luft an den Elektrodenkolben der UV-Lampe vorbei in ein Verbindungsrohr zur Aussenwand des Leitungsrohres gelangt, durch das die zu entkeimende Flüssigkeit fliesst. Die an der Aussenwand des Leitungsrohres sich abgekühlte Luft wird dann wiederum der UV-Lampe zugeführt und muss eine Temperatur aufweisen, die kleiner ist als die Betriebstemperatur des rohrförmigen Ansatzes. Die Einstellung der vorgebenen Temperatur wird mit Hilfe einer Heizung über den Fühler geregelt.

Um den Energiebedarf für die Aufheizung des rohrförmigen Absatzes zu senken, wird in der CH-A 636 479 vorgeschlagen, den Kühlstrom in zwei Teile aufzuteilen:

Ein Teilstrom wird unmittelbar zu dem Entladungsrohr der UV-Lampe geleitet; der andere, verglichen mit dem ersten geringeren Teilstrom wird durch die Heizung erwärmt und dem rohrförmigen Ansatz zugeleitet.

Einrichtungen nach den genannten schweizerischen Patentschriften haben sich in der Vergangenheit bei vielfältigen Applikationen hervorragend bewährt und leisteten einen wesentlichen Beitrag zur Betriebssicherheit und Erhöhung der Lebensdauer und damit Verfügbarkeit der mit diesen Lampen ausgerüsteten Anlagen.

Der Erfindung, wie sie in den Patentansprüchen gekennzeichnet ist, liegt die Aufgabe zugrunde, die bekannte Einrichtung zum Entkeimen einer Flüssigkeit im Hinblick auf ihren gesamten Energiebedarf zu optimieren und sie gleichzeitig für Anlagen mit einer grossen Anzahl von UV-Strahlen, wie sie z.B. in der Abwasserwirtschaft oder auch der Off-Shore-Technik benötigt werden, zu modifizieren.

Wesentliches Merkmal der Erfindung ist der Verzicht auf separate Heizeinrichtungen und die damit verbundenen individuellen Regelanordnungen zur Konstanthaltung der Temperatur des rohrförmigen Fortsatzes. Anstelle des Herunterkühlens des gesamten Kühlluftstromes oder auch nur eines Teilstromes auf Temperaturwerte unterhalb der optimalen Betriebstemperatur des rohrförmigen Fortsatzes und anschliessenden Wiederaufheizen nutzt die Erfindung sowohl in der Startphase als auch im regulären Betrieb konsequent die Verlustwärme der UV-Lampe selbst aus.

Wie dies im einzelnen bewerkstelligt wird und die sich aus dem neuen Kühlkonzept ergebenden Vorteile werden nachstehend anhand von in der Zeichnung dargestellten Ausführungsbeispielen näher erläutert.

In der Zeichnung zeigt:

Fig. 1 ein erstes Ausführungsbeispiel einer Einrichtung zur Entkeimung mit Innenkühlung im Querschnitt,

Fig. 2 ein zweites Ausführungsbeispiel mit Aussenkühlung,

Fig. 3 einen Querschnitt durch eine Entkeimungsanlage mit einer Vielzahl UV-Lampen,

Fig. 4 einen Horizontalschnitt durch die Anlage gemäss Fig. 3 längs deren Linie AA.

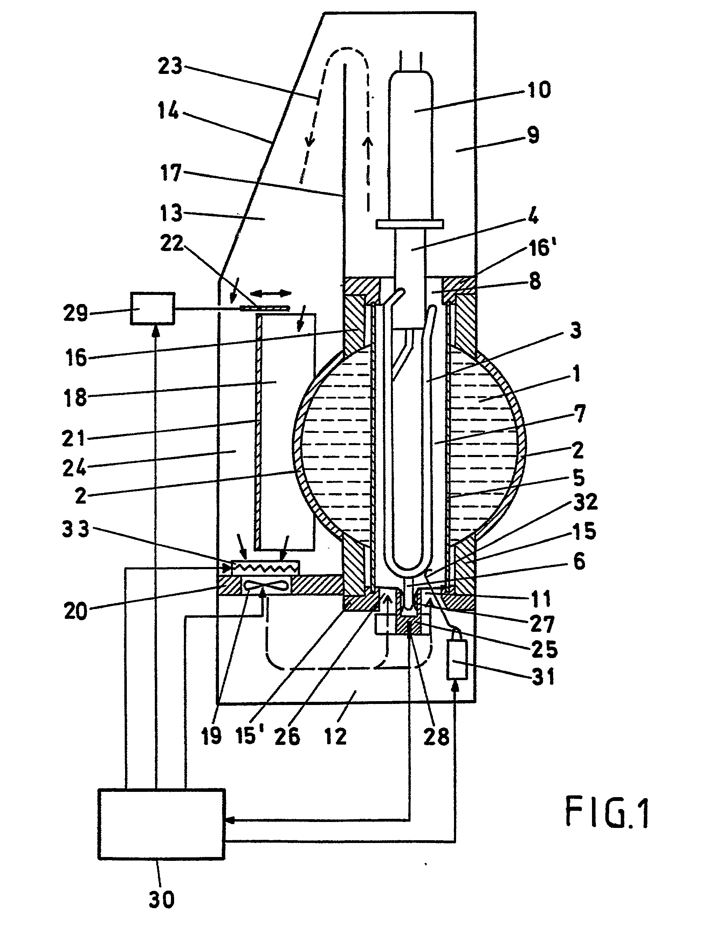

In Fig. 1 strömt das zu entkeimende Medium 1 durch ein Leitungsrohr 2, in dem ein beispielsweise U-förmiges Entladungsrohr 3 einer UV-Lampe 4 in einem Quarzschutzrohr 5 angeordnet ist. Am unteren Ende des Entladungsrohres 3 ist ein rohrförmiger Ansatz 6. Ein erster lufterfüllter Raum 7, welcher das Entladungsrohr 3 umgibt, weist eine Oeffnung 8 zu einem darüberliegenden zweiten lufterfüllten Raum 9 auf, welcher den Elektrodenkolben 10 der UV-Lampe 4 umgibt. Der Raum 7 ist über eine weitere Oeffnung 11 mit einem dritten lufterfüllten Raum 12 verbunden.

Die Räume 9 und 12 sind über einen Verbindungskanal 13 miteinander verbunden. Dieser Kanal ist gebildet durch die eine Hälfte eines die UV-Lampe 4 umgebenden Gehäuses 14, durch das in diesen Kanal 13 hineinragende Leitungsrohr 2 samt seinen Tragteilen 15, 16 sowie eine bis an das obere Ende des Röhrenkolbens 10 reichende Trennwand 17. Am Leitungsrohr 2 sind Kühlbleche 18 befestigt, welche in den Kanal 13 hineinragen.

Unmittelbar unterhalb der Kühlbleche 18 ist ein Ventilator 19 angeordnet, der in einem Schott 20 befestigt ist. Somit steht der Raum 12 nur über den Ventilatorquerschnitt mit dem Kanal 13 in Verbindung.

Die Kühlbleche 18 sind am gehäuseseitigen Ende mit einer Trennwand 21, die von der Gehäusewand 14 beabstandet ist, abgedeckt. Ein Schieber 22 ermöglicht es, die Luftströmung 23 - angedeutet durch Pfeile - im Kanal 13 einmal nur durch die Distanzen zwischen den Kühlblechen 18 oder nur durch den zwischen Trennwand 21 und Gehäusewand 14 gebildeten Umluftkanal 24 zu lenken, wobei in Zwischenstellung des Schiebers 22 beide Strömungswege mehr oder weniger beaufschlagt werden können.

Der rohrförmige Ansatz 6 taucht mit dem überwiegenden Teil seiner Länge in die Innenräume eines topfförmigen metallischen Wärmeübertragungskörpers 25, im folgenden Thermoblock genannt, ein. Dieser ist am unteren Ende des Quarzschutzrohrs 5 mittels speicherartigen Haltearmen 26 befestigt, die einstückig mit dem Thermoblock 25 ausgebildet sein können und den Wärmeübergang zwischen der Umgebung und dem Thermoblock verbessern. Der Aussendurchmesser des Thermoblocks 25 ist kleiner als die lichte Weite des Quarzschutzrohres, so dass er den Strömungsquerschnitt der Kühlluft durch das Rohr 5 nicht wesentlich einengt. Sein Innendurchmesser ist grösser als der Aussendurchmesser des rohrförmigen Ansatzes 6. Der verbleibende Ringspalt kann zusätzlich mit einer Wärmebrücke, die z.B. als Kontaktfeder 27 ausgebildet ist, versehen sein, um den Wärmeübergang vom Thermoblock 25 zum rohrförmigen Ansatz 6 zu verbessern.

In einer axialen Bohrung an der Unterseite des Thermoblocks 25 ist ein Temperatursensor 28 angeordnet, welcher - ebenso wie der Antrieb 29 des Schiebers 22 und der Ventilator 19 mit einer Steuer- und Regeleinrichtung 30 in Wirkverbindung steht.

Der Vollständigkeit halber ist in Fig. 1 auch die Hochspannungs-Zündeinrichtung 31 mit ihrer Zündelektrode 32 eingezeichnet.

Beim Betrieb der Einrichtung sind im wesentlichen zwei Phasen, die Startphase (bei kalter UV-Lampe) und die reguläre Betriebsphase zu unterscheiden.

Bevor die UV-Lampe gezündet werden darf, muss zunächst die Kathode (im Röhrenkolben 10) aufgeheizt werden. Diese Kathodenabwärme (typisch 40 - 60 W pro UV-Lampe) heizt die Luft im Raum 9 auf. Wird der Ventilator 19 zugeschaltet, und der Schieber 22 gleichzeitig in die rechte Endlage bewegt, stellt sich eine Luftzirkulation im Gehäuse 14 ein vom Raum 9 über den Verbindungskanal 13, durch den Umluftkanal 24, den Raum 12 in den Raum 7 zurück zum Raum 9 mit der "Wärmequelle" in Gestalt des Röhrenkolbens. Weil im Zuge dieses beschriebenen Strömungsweges - abgesehen von den Gehäusewänden 14 keine "Kühlflächen" liegen, wird das gesamte Gehäuseinnere und, was von wesentlicher Bedeutung ist, auch das u-förmige Entladungsrohr 3 samt dem rohrförmigen Ansatz 6 aufgeheizt. Erreicht die Temperatur der umgewälzten Luft den für die Zündung der UV-Lampe 4 optimalen Wert, typisch 60 °C, wird die UV-Lampe 4 gezündet.

Aufgrund der engen thermischen Kopplung des rohrförmigen Ansatzes 6 über den Thermoblock 25 mit dem darin angeordneten Temperatur-Sensor weist auch der Ansatz diese Temperatur auf.

In der sich nun anschliessenden regulären Betriebsphase heizt die UV-Lampe - ihr Wirkungsgrad liegt aus physikalischen Gründen nur bei etwa 30 % - die durch den Raum 7 strömende Luft auf. Sie gelangt auf dem oben beschriebenen Wege zurück zum Thermoblock 25. Ueberschreitet die Temperatur einen einstellbaren Wert, im Beispielsfall 60 °C, so wird mittels der Steuer- und Regeleinrichtung 30 entweder die Förderleistung des Ventilators 19 erhöht und/oder der Schieber nach links bewegt, welcher damit den Weg durch den Kühler (Kühlbleche 18 in Verbindung mit dem Wärmetausch-Medium 1) öffnet. Die Zahl der Kühlbleche 18 und ihre Dimensionierung sowie die Abmessungen der Fläche des Leitungsrohres 2, die mit der zu kühlenden Luft in Berührung kommt, sind derart gewählt, dass auch bei Vollast der UV-Lampe die Temperatur der Luft am Thermoblock 25 nicht den vorgegebenen Wert überschreitet.

Sollten es die Gegebenheiten erfordern, dass die Aufheizung der Kühlluft in der Startphase allein durch die Kathodenabwärme zu lange dauert, oder die Abwärme nicht ausreicht, kann im Kanal 13 eine nur in der Startphase zuschaltbare, ansonsten ungeregelte Zusatzheizung 33, z.B. unmittelbar vor dem Ventilator 19 auf dem Schott 20 vorgesehen werden. Diese wird jedoch unmittelbar vor dem Zünden der UV-Lampe, möglicherweise schon vorher, wieder abgeschaltet.

Das beschriebene System garantiert die Einhaltung der für den Quecksilber-Dampfdruck - und damit auch für die Strahlungsintensität verantwortliche Solltemperatur am rohrförmigen Ansatz 6 auch unter variierenden Betriebs(Temperatur-)Verhältnissen. Vor allem kann der Kühlluftstrom zur Verbesserung der Strahlerkühlung ohne zusätzlichen Energieaufwand auf das Mehrfache gesteigert werden, ohne Gefahr zu laufen, dass die Temperatur des Strahlers am Eintritt der Kühlluft unter die Solltemperatur des rohrförmigen Fortsatzes 6 fällt.

Diese Erhöhung des Kühlluftdurchsatzes bringt - entgegen der Anordnung nach dem CH-Patent 636 479 - auch nicht die Gefahr mit sich, dass dieser unterhalb der Solltemperatur des rohrförmigen Ansatzes temperierte Luftstrom den sich an den Ansatz anschliessenden unteren Abschnitt des Entladungsrohrs 3 unterkühlt, was insbesondere in der Startphase zur Beschädigung wenn nicht gar zur Zerstörung der UV-Lampe führen kann.

Ein weiterer Vorteil der Erfindung ist darin zu sehen, dass der Thermoblock 25 den rohrförmigen Ansatz 6 vom Entladungsrohr 3 thermisch abschirmt, die Strahlungswärme der Entladung somit nicht den rohrförmigen Ansatz 6 aufheizt. Gleichzeitig wirkt er aber auch als Wärmepuffer, so dass ein kurzfristiges Abfallen der Kühllufttemperatur keine unmittelbare Auswirkung auf die Temperatur am Ansatz 6 hat. Auch in elektrischer/magnetischer Hinsicht erfüllt der Thermoblock eine Abschirmfunktion gegenüber dem Temperatur-Sensor 28, weil durch ihn von der Entladung herrührende elektrische und magnetische Felder vom Temperatur-Sensor 28 ferngehalten werden, die ihrerseits störend auf die Temperaturregelung wirken können.

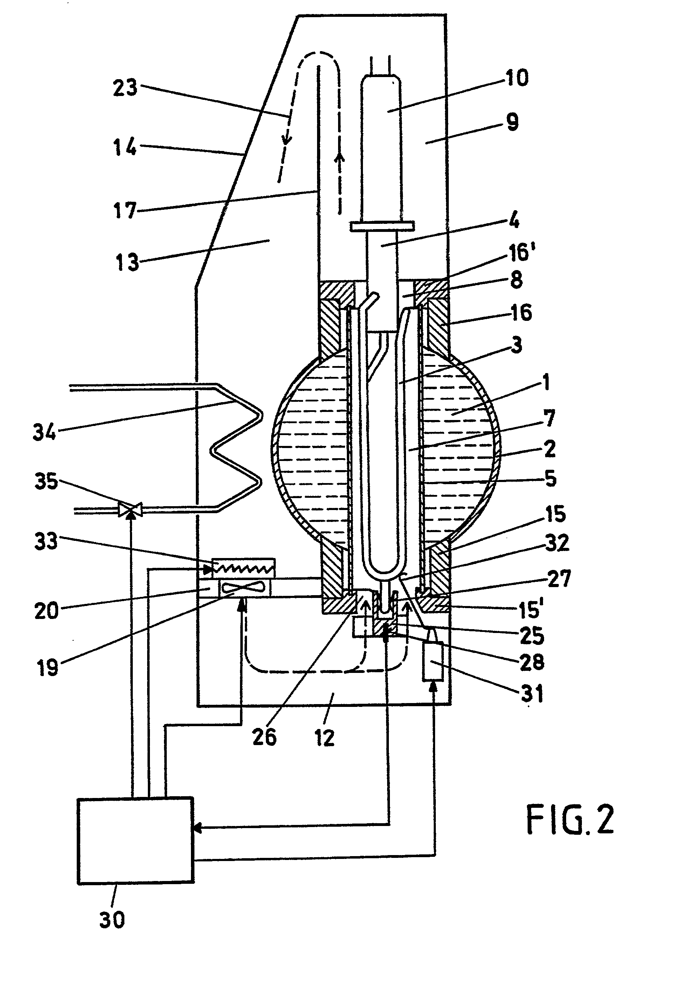

Wie in Fig. 2 beispielsweise veranschaulicht ist - in dieser sind gleiche Teile wie in Fig. 1 mit denselben Bezugszeichen versehen - lässt sich die Erfindung auch mit Fremd- oder Aussenkühlung realisieren. Die Kühlbleche 18, die Trennwand 21 und damit der Umluftkanal 24 sowie der Schieber 22 entfallen. An deren Stelle tritt ein Kühler 34 im Kanal 13, der über ein Ventil 35, das mit der Steuer- und Regeleinrichtung 30 in Wirkverbindung steht, zuschaltbar ist.

Die Wirkungsweise der Einrichtung gemäss Fig. 2 entspricht prinzipiell derjenigen nach Fig. 1. Der Kühler 34 und/oder der Ventilator 19 werden in Abhängigkeit der am Thermoblock 25 erfassten Temperatur ein- bzw. ausgeschaltet bzw. gesteuert.

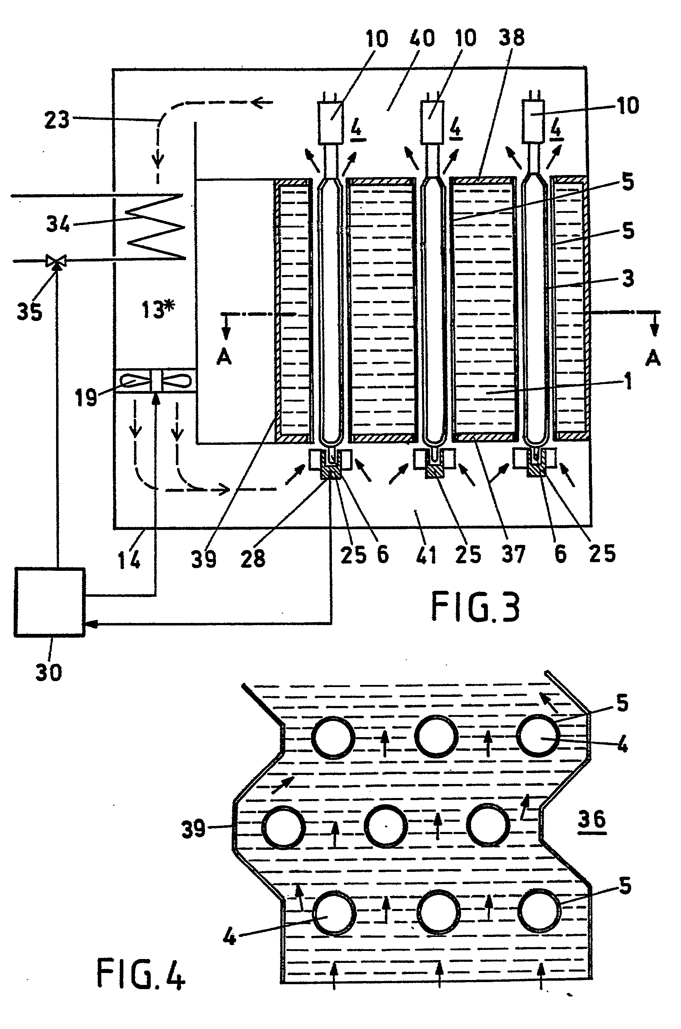

Gegenüber beiden bekannten Einrichtungen (CH-A 631 950 und 636 479), bei denen jeder UV-Lampe eine eigene Temperaturregelung zugeordnet sein muss, ist es bei der Ausführung nach Fig. 2 überdies möglich, mehreren UV-Lampen einen gemeinsamen Kühler mit einer gemeinsamen Temperaturregelung mit nur einem Temperatur-Sensor 28 an einem einzigen Thermoblock zu realisieren. Dies ist in den Figuren 3 und 4 beispielsweise vereinfacht veranschaulicht.

Die UV-Lampen 4 sind in einem Kanal 36 mit Rechteckquerschnitt in Reihen hintereinander und gegeneinander versetzt angeordnet. Der Kanal 36 ist durch Bodenplatten 37 und Deckplatten 38 sowie Seitenwände 39 begrenzt. Die Quarzschutzröhren 5 sind in entsprechend ausgebildeten Bohrungen in Boden- und Deckplatte flüssigkeitsdicht eingesetzt. Die zu den Lampenachsen parallelen Seitenwände 39 sind derart gewellt (Fig. 4), dass der Strömungsquerschnitt für das zu entkeimende Medium 1 überall annähernd gleich ist und sich infolge der guten Durchmischung eine gleichmässige Bestrahlung des gesamten Flüssigkeitsstromes erreichen lässt. Die Röhrenkolben 10 der UV-Lampe ragen oben in einen Anströmraum 40, der von den Deckplatten 38 und der oberen Gehäusewand 14 begrenzt ist.

Die rohrförmigen Ansätze 6 der Entladungsrohre 3 sind analog Fig. 1 und 2 von den Thermoblöcken 25 umgeben und ragen in einen Einströmraum 41 hinein, der durch die Bodenplatte 37 und die untere Gehäusewand 14 begrenzt ist. Ausströmraum 40 und Einströmraum 41 sind über einen Kanal 13* verbunden, der durch die seitliche Gehäusewand und die Seitenwand 39 begrenzt ist. In diesem Kanal ist der Kühler 34 und der Ventilator 19 analog Fig. 2 angeordnet.

Nur an einem Thermoblock 25 ist ein Temperatur-Sensor 28 angeordnet, der mit der Steuer- und Regeleinrichtung in Wirkverbindung steht. Die Wirkungsweise der Einrichtung entspricht derjenigen nach Fig. 2.

Umfangreiche Messungen haben ergeben, dass die Durchmischung der Kühlluft im Einströmraum 41 so vollkommen ist, dass an allen Thermoblöcken 25 und damit an allen rohrförmigen Ansätzen 6 praktisch die gleiche Temperatur herrscht.

Auch in der Startphase führt die vergleichsweise massive Ansammlung von Wärmequellen in Gestalt der Röhrenkolben 10 im Ausströmraum 40 zu einer schnellen Aufheizung der Kühlluft, so dass gegebenenfalls auf eine Zusatzheizung verzichtet werden kann.

**Patentansprüche**

1. Einrichtung zum Entkeimen von Flüssigkeiten mittels ultravioletter Strahlen, mit einer Niederdruck-Hochstrom-Quecksilberdampflampe (4), die einen am Entladungsrohr (3) angeordneten rohrförmigen Ansatz (6) und einen von diesem distanzierten, die Kathodenheizung enthaltenden Röhrenkolben (10) aufweist, mit einem ersten Raum (7), welcher das Entladungsrohr (3) umgibt, der seinerseits vom Behandlungsraum (2) für die zu entkeimende Flüssigkeit (1) umgeben ist, welcher erste Raum (7) eine erste Oeffnung (8) zu einem zweiten Raum (9) aufweist, der den Röhrenkolben (10) umgibt, sowie am rohrförmigen Ansatz (6) eine zweite Oeffnung (11) an einem dritten Raum (12), wobei der zweite (9) und dritte Raum (12) über einen Verbindungskanal (13) miteinander verbunden sind, und eine Kühlfläche (18; 34) und einen Ventilator (19) aufweisenden Kühleinrichtung, dadurch gekennzeichnet, dass der rohrförmige Ansatz (6) zumindest teilweise von einem metallischen Wärmeleitkörper (25) umgeben ist, der zumindest teilweise in den dritten Raum (12) hineinragt, dass am Wärmeleitkörper (25) ein Temperatur-Sensor (28) angeordnet ist, der mit einer Steuer- und Regeleinrichtung (30) in Wirkverbindung steht, dass an die besagte Steuer- und Regeleinrichtung ferner Mittel (22; 35) zur Steuerung des Kühlmitteldurchsatzes durch den genannten Kühler (18; 34) vorgesehen sind, um die Temperatur am Wärmeleitkörper (25) auf eine vorgegebene Temperatur zu regeln.

2. Einrichtung nach Anspruch 1, dadurch gekennzeichnet, dass der Wärmeleitkörper (25) topfförmig ausgebildet ist, wobei das freie Ende des rohrförmigen Ansatzes (6) zumindest teilweise in das Topfinnere eintaucht.

3. Einrichtung nach Anspruch 2, dadurch gekennzeichnet, dass zwischen Topfinnenwand und rohrförmigem Ansatz (6) eine Wärmebrücke (27) vorgesehen ist.

4. Einrichtung nach einem der Ansprüche 1 bis 3, dadurch gekennzeichnet, dass am Behandlungsraum (2) in den Verbindungskanal (13) ragende Kühlbleche (18) angeordnet sind, die seitlich durch eine Trennwand (21) abgedeckt sind, derart, dass zwischen der Trennwand (21) und einer Gehäusewand (14) ein Umluftkanal (24) ausgebildet ist, dass eine Leiteinrichtung (22) zum wahlweisen Verschliessen des Umluftkanals (24) bzw. Oeffnen des Strömungsweges durch die Distanzen zwischen den Kühlblechen (18) in Abhängigkeit von der Temperatur am Wärmeleitkörper (6) vorgesehen ist.

5. Einrichtung nach einem der Ansprüche 1 bis 3, dadurch gekennzeichnet, dass im Verbindungskanal (13) ein Kühler (34) und Mittel (35) zur Steuerung der Kühlleistung des Kühlers (34) in Abhängigkeit von der Temperatur am Wärmeleitkörper (6) vorgesehen sind.

6. Einrichtung nach einem der Ansprüche 1 bis 5, dadurch gekennzeichnet, dass im Verbindungskanal (13) eine nur in der Startphase zuschaltbare Wärmequelle (33) vorgesehen ist.

7. Einrichtung nach einem der Ansprüche 1 bis 6, gekennzeichnet durch Anordnung einer Mehrzahl von UV-Strahlern (4) in einem gemeinsamen Gehäuse (14), wobei allen UV-Strahlern eine gemeinsame Kühleinrichtung (34) zugeordnet ist, deren Kühlwirkung durch einen einzigen Temperatur-Sensor (28) über die Steuer- und Regeleinrichtung (30) kontrollierbar ist.

8. Einrichtung nach Anspruch 7, dadurch gekennzeichnet, dass das Gehäuse einen Behandlungsraum (36) für die zu entkeimende Flüssigkeit, einen Einströmraum (41) und einen Ausströmraum (40) für das Kühlmedium sowie einen die beiden letztgenannten Räume verbindenden Kanal (13*) umfasst, wobei die Röhrenkolben (10) in den Ausströmraum (40) und die von den Wärmeleitkörpern (25) umgebenen rohrförmigen Ansätzen (6) in den Einströmraum (41) hineinragen.

9. Einrichtung nach Anspruch 8, dadurch gekennzeichnet, dass der Behandlungsraum (36) durch Boden- (37), Deckplatten (38) und Seitenwände (39) begrenzt ist, die Boden- und Deckplatten (37, 38) weisen kongruente Bohrungen auf, in welchen die Quarzschutzrohre (5) der UV-Strahler flüssigkeitsdicht verfestigt sind.

10. Einrichtung nach Anspruch 9, dadurch gekennzeichnet, dass die einzelnen UV-Strahler (4) in Reihen hintereinander und gegeneinander versetzt angeordnet sind und die zu den Strahlerachsen parallelen Seitenwände (39) gewellt sind.

**Claims**

1. Apparatus for sterilizing liquids by means of ultraviolet rays, with a low-pressure high-current mercury-vapour lamp (4) which has a tubular extension (6) arranged on the discharge tube (3) and a tube bulb (10) located at a distance from the said tubular extension (6) and containing the cathode heating system, and with a first space (7) surrounding the discharge tube (3), itself surrounded by the treatment space (2) intended for the liquid (1) to be sterilized, the said first space (7) having a first orifice (8) to a second space (9) surrounding the tube bulb (10) and, at the tubular extension (6), a second orifice (11) to a third space (12), the second space (9), and the third space (12) communicating with one another via a connecting duct (13), and a cooling device having a cooling surface (18; 34) and a fan (19), characterized in that the tubular extension (6) is surrounded at least partially by a metal heat-conducting body (25) which projects at least partially into the third space (12), in that arranged on the heat-conducting body (25) is a temperature sensor (28) which is connected operatively to a control and regulating device (30), and in that there are also, in the said control and regulating device, means (22; 35) of controlling the throughput of cooling medium through the said cooler (18; 34 ), in order to regulate the temperature on the heat-conducting body (25) to a predetermined temperature.

2. Apparatus according to Claim 1, characterized in that heat-conducting body (25) is made pot-shaped, the free end of the tubular extension (6) penetrating at least partially into the pot interior.

3. Apparatus according to Claim 2, characterized in that heat bridge (27) is provided between the pot inner wall and the tubular extension (6).

4. Apparatus according to one of Claims 1 to 3, characterized in that cooling plates (18) projecting into the connecting duct (13) are arranged on the treatment space (2) and are covered laterally by means of a partition wall (21), in such a way that a circulation duct (24) is formed between the partition wall (21) and a housing wall (14), and in that there is a guide device (22) for selectively closing the circulation duct (24) and opening the flowpath through the gaps between the cooling plates (18) as a function of the temperature on the heat-conducting body (6).

5. Apparatus according to one of Claims 1 to 3, characterized in that a cooler (34) and means (35) of controlling the cooling capacity of the cooler (34) as a function of the temperature on the heat-conducting body (6) are provided in the connecting duct (13).

6. Apparatus according to one of Claims 1 to 5, characterized in that heat source (33) which can be cut in only in the starting phase is provided in the connecting duct (13).

7. Apparatus according to one of Claims 1 to 6, characterized in that a plurality of ultraviolet lamps (4) is arranged in a common housing (14), all the ultraviolet lamps having assigned to them a common cooling device (34), the cooling capacity of which can be controlled by a single temperature sensor (28) via the control and regulating device (30).

8. Apparatus according to Claim 7, characterized in that the housing comprises a treatment space (36) for the liquid to be sterilized, an inflow space (41), and an outflow space (40) for the cooling medium and a duct (13*) connecting the two last-mentioned spaces, the tube bulbs (10) projecting into the outflow space (40) and the tubular extensions (6) surrounded by the heat-conducting bodies (25) projecting into the inflow space (41).

9. Apparatus according to Claim 8, characterized in that the treatment space (36) is limited by bottom plates (37), cover plates (38) and side walls (39), the bottom and cover plates (37, 38) having congruent bores, in which the quartz guard tubes (5) of the ultraviolet lamps are fastened in a liquid-tight manner.

10. Apparatus according to Claim 9, characterized in that the individual ultraviolet lamps (4) are arranged successively in rows and offset relative to one another, and the side walls (39) parallel to the lamp axes are corrugated.

## Revendications

1. Dispositif pour stériliser des liquides au moyen de rayonnements ultraviolets, avec une lampe à vapeur de mercure (4) à haute intensité et basse pression, qui présente un raccord tubulaire (6) monté sur le tube à décharge (3) et une ampoule de tube (10) distante de ce raccord et contenant le chauffage par cathode, avec un premier volume (7) qui entoure le tube à décharge (3) et qui est à son tour entouré par un volume de traitement (2) pour le liquide à stériliser (1), premier volume (7) qui présente une première ouverture (8) vers un deuxième volume (9) qui entoure l'ampoule de tube (10), ainsi qu'au raccord tubulaire (6) une deuxième ouverture (11) vers un troisième volume (12), le deuxième volume (9) et le troisième volume (12) étant reliés par un canal de liaison (13), et un dispositif de refroidissement comprenant une surface de refroidissement (18; 34) et un ventilateur (19), caractérisé en ce que le raccord tubulaire (6) est entouré au moins partiellement par un corps métallique (25) conducteur de la chaleur qui pénètre au moins partiellement dans le troisième volume (12), en ce que le corps conducteur de la chaleur (25) est pourvu d'un capteur de température (28) qui se trouve en liaison active avec un dispositif de commande et de régulation (30), en ce qu'audit dispositif de commande et de régulation il est en outre prévu des moyens (22; 35) de commande du débit du fluide de refroidissement à travers ledit refroidisseur (18; 34), afin de réguler la température du corps conducteur de la chaleur (25) à une température prédéterminée.

2. Dispositif suivant la revendication 1, caractérisé en ce que le corps conducteur de la chaleur (25) a la forme d'un pot, l'extrémité libre du raccord tubulaire (6) plongeant au moins partiellement à l'intérieur du pot.

3. Dispositif suivant la revendication 2, caractérisé en ce qu'il est prévu un pont thermique (27) entre la paroi intérieure du pot et le raccord tubulaire (6).

4. Dispositif suivant l'une ou l'autre des revendications 1 à 3, caractérisé en ce qu'au volume de traitement (2), il est prévu des tôles de refroidissement (18) plongeant dans le canal de liaison (13), qui sont couvertes par une paroi de séparation (21), de telle sorte qu'il se forme un canal de dérivation de l'air (24) entre la paroi de séparation (21) et une paroi d'une enceinte (14), et en ce qu'il est prévu un dispositif de direction (22) pour sélectivement fermer le canal de dérivation de l'air (24) respectivement ouvrir le chemin d'écoulement à travers les espaces entre les tôles de refroidissement (18) en fonction de la température du corps conducteur de la chaleur (25).

5. Dispositif suivant l'une ou l'autre des revendications 1 à 3, caractérisé en ce que, dans le canal de liaison (13), il est prévu un refroidisseur (34) et des moyens (35) pour commander la puissance de refroidissement du refroidisseur (34) en fonction de la température du corps conducteur de la chaleur (25).

6. Dispositif suivant l'une ou l'autre des revendications 1 à 5, caractérisé en ce que, dans le canal de liaison (13), il est prévu une source de chaleur (33) qui ne peut être mise en service que pendant la phase de démarrage.

7. Dispositif suivant l'une ou l'autre des revendications 1 à 6, caractérisé par le placement d'une pluralité d'émetteurs de rayonnements UV dans une enceinte commune (14), l'ensemble des émetteurs de rayonnements UV étant associé à un dispositif de refroidissement (34) commun, dont l'action refroidissante peut être commandée par un seul capteur de température (28) par l'intermédiaire d'un dispositif de commande et de régulation (30).

8. Dispositif suivant la revendication 7, caractérisé en ce que l'enceinte englobe un volume de traitement (36) pour le liquide à stériliser, un volume d'entrée (41) et un volume de sortie (40) pour le fluide de refroidissement ainsi qu'un canal (13*) reliant les deux volumes précités, les ampoules de tube (10) pénétrant dans le volume de sortie (40) et les raccords tubulaires (6) entourés par les corps conducteurs de la chaleur (25) pénétrant dans le volume d'entrée (41).

9. Dispositif suivant la revendication 8, caractérisé en ce que le volume de traitement (36) est limité par des plaques inférieures (37), des plaques supérieures (38) et des parois latérales (39), et en ce que les plaques inférieures et supérieures (37, 38) présentent des trous congruents dans lesquels sont hermétiquement fixés les tubes de protection en quartz (5) des émetteurs de rayonnements UV.

10. Dispositif suivant la revendication 9, caractérisé en ce que les émetteurs de rayonnements UV individuels (4) sont disposés en série l'un derrière l'autre et sont déportés l'un par rapport à l'autre, et en ce que les parois latérales (39) parallèles à l'axe des émetteurs sont ondulées.

EP 0 236 575 B1

FIG.1

FIG.2

FIG.3

FIG.4